# EUROPEAN PATENT SPECIFICATION

(11) **EP 0 969 083 B1**
(45) Date of publication and mention of the grant of the patent: **24.03.2004**
(21) Application number: 98940614.5
(22) Date of filing: 28.08.1998
(51) Int. Cl.: C12M 1/00, C12N 15/09, C12Q 1/68, B01L 3/00, B01J 19/00

(54) **DNA CAPILLARY**
DNA KAPILLARE
CAPILLAIRE D'ADN

(30) Priority: 29.08.1997 JP 23414597
(43) Date of publication of application: 05.01.2000
(73) Proprietor: Olympus Optical Co., Ltd., Tokyo 151-0072 (JP)
(72) Inventor: SUYAMA, Akira, Hachioji-shi Tokyo 192-0372 (JP)
(74) Representative: KUHNEN & WACKER
(86) International application number: PCT/JP1998/003852
(87) International publication number: WO 1999/011754

(56) References cited:
- EP-A- 0 430 248
- WO-A-82/02211
- WO-A-92/04613
- JP-T- 7 505 529
- JP-T- 7 506 561

## Description

### Technical Field

The present invention relates to an apparatus for detecting a target DNA, a target mRNA, etc. by using a DNA probe.

### Background Art

In recent years, a human genome project, i.e., an attempt to analyze the base sequence in all the human genes, is being carried out worldwide. The human genome analysis including the determination of the base sequence is a very complex and laborious work. It is said, however, that the human genome project will be completed at the beginning of the 21st century. Development of many novel technologies as well as improvement and automation of the analytical equipment greatly contributes to the promotion of the human genome project. A DNA chip technology is one of the newly developed analytical technologies.

The DNA chip is a chip prepared by spotting many kinds of DNA probes at predetermined positions on a substrate, e.g., a silicon wafer, by utilizing the lithographic technology used in the field of semiconductor devices. The DNA probe is formed of an oligonucleotide having a predetermined sequence of 4 kinds of bases constituting DNA, i.e., adenine (A), guanine (G), cytosine (C) and thymine (T). The sequence is complementary to the base sequence of the target DNA or mRNA. For example, if the sequence of AGCTT (5'→3') is used as a DNA probe, a DNA having a base sequence of AAGCT, which is complementary to the sequence of AGCTT noted above, is hybridized with the DNA probe so as to be selectively caught. Incidentally, the actual constituting unit of the DNA probe is nucleotide having the above-noted base portion (base portion + deoxyribose portion + phosphoric acid ester portion). For simplifying the description, however, nucleotide is represented by the base alone in the following description.

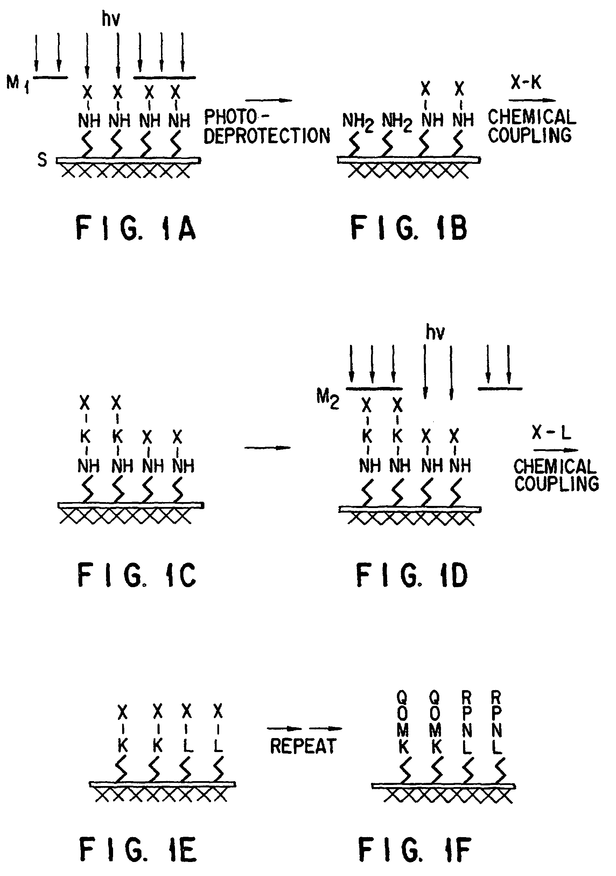

A method of immobilizing a DNA probe on the substrate is exemplified in, for example, "Science 251:767-773" published in February 1991. In this method, a DNA probe is formed on a flat substrate by utilizing a photochemical reaction. Let us describe briefly how to form a DNA probe having a 4-base length on a silicon substrate by this method with reference to FIG. 1.

In the first step, an amino group is formed on a silicon substrate S by treatment with silane, followed by allowing a photo-protective group X to be coupled with each amino group. Then, a desired position is selectively irradiated with an ultraviolet light by using a first mask M₁. As a result, a protective group x at a desired position is removed so as to expose the amino group, as shown in FIG. 1A. Then, an optional DNA base (shown by K here) accompanied by the photolabile protecting group is reacted with the exposed amino group, as shown in FIG. 1B. As a result, a portion in which the base K accompanied by the photolabile protecting group X is coupled with the amino group and another portion in which the photolabile protecting group X alone is coupled with the amino group are formed on the substrate, as shown in FIG. 1C. Further, the portion in which the photolabile protecting group X alone is coupled with the amino group is selectively irradiated with light through a second mask M₂ so as to selectively remove the photolabile protecting group X in the irradiated portion. Then, an optional DNA base (shown by L here) accompanied by the photolabile protecting group X is coupled with the exposed amino group, as shown in FIG. 1D. As a result, bases K and L accompanied by the photolabile protecting group X are immobilized to the substrate surface with an amino group (not shown) interposed therebetween, as shown in FIG. 1E. Further, similar operations are performed by using an optional DNA base (shown by M here) and another optional DNA base (shown by N here). As a result, a DNA probe having a length of two bases is immobilized to the substrate surface. Still further, similar operations are repeated to stack bases in a three dimensional direction so as to form DNA probes each having a length of four bases, said DNA probes having different base sequences depending on the process units, as shown in FIG. 1F. For forming a base sequence having a length of, for example, eight bases, photolithography using 32 masks and the photoreaction are repeated 32 times so as to form DNA probes having all the desired base sequences. It is theoretically possible to form efficiently DNA probes each having an optional base length and an optional base sequence on a single substrate by utilizing the above-described technology.

An improved method of manufacturing a DNA chip is disclosed in International Laid-open Application WO 93/096668 (Japanese translation version No. 7-506561). In this method, a DNA probe is immobilized on a silicon substrate having an amino group formed thereon by using a flow type channel block. The flow type channel block used in this method is a pattern plate having a plurality of slender channels. In the case of using the pattern plate, it is possible to immobilize the DNA probe along each of these channels. In this case, the base sequence of the DNA probe differs depending on the channel. However, the base sequence is the same over the entire length in a single channel. In this preferred mode of the conventional technology, an amino group is attached first to the substrate, followed by combining the substrate with a block having a plurality of channels arranged in parallel. Then, a process solution containing a base, which is a constituting unit of the selected DNA probe, is allowed to flow through a predetermined channel so as to immobilize the first base of the aimed DNA probe. Further, the substrate and the channel block are rotated relative to each other by a predetermined angle, e.g., 90°, followed by combining again the substrate and the channel block and subsequently immobilizing a base corresponding to the second base along the channel. A DNA probe having a desired base sequence can be prepared by successively repeating the above-noted steps. Also, a large number of DNA chips referred to previously can be manufactured in a single operation by combining the method described above with the photochemical reaction. It should also be noted that screening can be performed by using the DNA chip prepared by the above-noted method in combination with the channel block described above.

In order to prepare the conventional DNA chip, it is necessary to carry out mutual reactions among a large number of reagents on a flat substrate in immobilizing the probe. Also, when the DNA chip is used for measurement, it is necessary to apply many times a liquid material to a surface of a flat DNA chip in order to carry out reactions with a sample and to wash the DNA chip surface. In order to apply the above treatment to a flat substrate (or DNA chip), the substrate (or the DNA chip) must be dipped in a reactant solution filling a container. Alternatively, a flow path must be formed on the DNA chip surface by using an additional tool such as the channel block noted above, followed by applying the treatment with the liquid. However, the dipping method gives rise to a problem that each kind of the treating liquid must be used in an excessive amount in the immobilizing step and in the sample measuring step. On the other hand, in the method using a flow path, only a limited region of the surface of the DNA chip having a DNA probe immobilized thereon is used, resulting in failure to utilize sufficiently the formed DNA chip. Further, the DNA chip is an open system in which the probe-formed surface is exposed to the outside, giving rise to the defects that the surface tends to be contaminated and that the DNA chip cannot be handled conveniently.

The conventional DNA chip technology gives rise to an additional problem besides the problems given above. Specifically, the conventional DNA chip technology is certainly effective for performing the sequencing of DNA having an unknown base sequence, but is not adapted for the developed pattern analysis of mRNA which will be important in the future. Let us describe the particular problem in the following.

In recent researches on genes; how to utilize the DNA information obtained by the sequencing is more important in view of the post genome than the sequencing itself. For example, the analysis of the mRNA expression pattern is being carried out vigorously in order to make researches on the gene expression profile. The expression of mRNA relating to a certain gene exhibits a different level of expression depending on each organ. Even in the same cell line, the level of expression differs depending on phase factors, particular disease factors, etc. The analysis of the difference in the level of the mRNA expression in an individual, i.e., analysis of the expression pattern, can be applied in various technical fields such as gene diagnosis, gene therapy, development of medicines and agricultural and stock raising industries and, thus, a further progress is expected.

In general, a quantitative analysis of mRNA relating to a target gene is required for the analysis of the expression pattern of mRNA. Employed for the quantitative analysis is a method in which mRNA to be measured or cDNA thereof, which is obtained by a reverse transcription from mRNA, is reacted with a DNA probe having a base sequence complementary thereto so as to hybridize both of them and, thus, to achieve detection. In such a research, a plurality of expression levels of mRNA are measured. Therefore, used are a plurality of DNA probes corresponding to these plural target mRNA molecules. It should also be noted that, in a research on the expression pattern of mRNA, the base sequence of the mRNA or cDNA to be measured is known to some extent, making it possible to use a relatively long DNA probe having about 20 to 60 bases, preferably, about 40 bases, in view of the accuracy and efficiency of the measurement. As a matter of fact, such a relatively long DNA probe is actually used. In order to prepare a probe having such a length by the conventional DNA chip technology, a tremendous labor is required such that it is necessary to prepare 80 to 240 masks and to repeat the base synthesis by lithography and photoreaction 80 to 240 times. Further, in order to improve the measuring accuracy, it is necessary to align the length of the DNA probe. In the case of employing the conventional method, it is substantially impossible to synthesize a DNA probe having an aligned base length directly on a substrate in view of the yield of synthesis in each stage.

On the other hand, about 20,000 to 30,000 kinds of genes of about 100,000 genes present in a single cell are considered to express mRNA. Since a ratio of cell specific genes that are important in the research of the expression pattern is estimated at about several percent (about 1 to 3%), about several hundred to thousand of mRNA or their cDNA are considered to be capable of providing a target object to be measured. According to the conventional DNA chip technology described previously, 4⁸ kinds, i.e., 65,536 kinds, of probes each having a length of 8 bases can be formed on a single substrate. However, since a maximum of about 20,000 to 30,000 kinds of mRNA, actually not larger than 1/10 thereof, are considered to be capable of providing the target object, such a tremendous number of kinds of probes need not be formed in practice.

Further, the hybridization condition between a DNA probe and a target object of an mRNA (or its cDNA) segment is not uniform and requires the stringency in the reaction. In addition, it is impossible in view of the dynamic range of the measuring apparatus to process in a single operation a test sample containing a tremendous number of kinds of target mRNA (or cDNA) pieces widely different from each other in concentration to detect the target mRNA. Therefore, even if a tremendous number of kinds of DNA probes are prepared under the same measuring conditions, only a very small proportion of the DNA probes produce a useful result. Such being the situation, a tremendous number of kinds of DNA probes need not be formed on the same substrate.

Further in the conventional DNA chip technology described previously, the synthesis by photoreaction must be repeated for forming the DNA probe, with the result that deterioration of the DNA probe caused by ultraviolet light remains as a problem to be solved. It follows that the conventional DNA chip technology is unsuitable for use in preparation of a DNA probe having a length of 20 bases or more.

### Disclosure of Invention

A first object of the present invention is to provide a DNA probe apparatus strong against contamination, easy to handle and excellent in efficiency.

A second object of the present invention is to provide a DNA probe apparatus which permits immobilizing the DNA probe without requiring excessive amounts of various processing liquids and also permits enlarging the area that can be utilized.

A third object of the present invention is to provide a DNA probe apparatus which permits detecting a plurality of target DNA, etc. at once.

Further, a fourth object of the present invention is to provide a DNA capillary adapted for analysis of the expression pattern of mRNA.

The first to fourth objects can be achieved by a DNA capillary, comprising a fluid passageway having at least a part thereof defined by a wall capable of transmitting light, a plurality of independent probe regions formed in the inner wall of the fluid passageway, and DNA probes immobilized on the probe regions, respectively, the DNA probes differing from each other depending on the probe regions on which the DNA probes are immobilized.

In an aspect of the present invention, the fluid passageway should desirably be a hollow capillary having the end portion left open. More desirably, the fluid passageway should be a cylindrical capillary. Where the fluid passageway is in the form of a cylinder having the end portion alone left open, the DNA capillary forms a closed system, making it possible to provide a DNA capillary very strong against contamination and easy to handle.

In another aspect of the present invention, a plurality of fluid passageways are arranged in an integral form so as to improve the immobilizing treatment of the DNA probe and the processing capability of the test sample. Particularly, where all of the plural fluid passageways are combined to communicate with each other in the vicinity of at least one end portion of the fluid passageway, the various processing liquids can be collectively introduced into or recovered from the plural fluid passageways through the combined portion.

In another aspect of the present invention, it is desirable to arrange the plural probe regions within a single fluid passageway in the form of annular regions apart from each other along the fluid passageway. The particular arrangement permits improving the efficiency because a plurality of different DNA probes are allowed to perform the detecting function simultaneously by simply passing liquid to be treated, which contains a test sample, or a gas for the drying purpose, through the single fluid passageway only once. Further, in the DNA capillary of the present invention, it is desirable for the different DNA probes to be arranged in annular regions formed over the entire circumferential region of the inner wall of the capillary and positioned apart from each other. The particular construction makes it possible to enlarge the area to be utilized, compared with the conventional DNA chip utilizing a flat plane. As a result, the target substance can be caught efficiently even in the case of using a small amount of a test sample.

In the DNA capillary of the present invention, the fluid passageway can be formed by applying an etching to a glass or silicon substrate. The etching method is desirable because a large number of capillaries can be prepared by a single processing. In addition, a large number of DNA probes can be immobilized within a large number of capillaries by utilizing a photochemical reaction.

What should also be noted is that it is desirable in the present invention to use a DNA probe synthesized in advance with a predetermined base sequence and length. In this case, it is possible to provide a DNA probe adapted for the analysis of the expression pattern of mRNA so as to achieve the fourth object of the present invention. To be more specific, since the probe can be immobilized by applying a photochemical reaction only once, it is possible to immobilize stably a DNA probe having at least 20 bases. It follows that it is possible to provide a detecting apparatus adapted for the research on the mRNA expression pattern, though it was impossible to achieve such a detecting apparatus by utilizing the conventional DNA chip technology.

### Brief Description of Drawings

FIGS. 1A to 1F schematically show a conventional method of immobilizing DNA probes.
FIG. 2 shows a DNA capillary according to a first embodiment of the present invention.
FIG. 3 schematically shows a method of immobilizing DNA probes on the inner wall of a capillary.
FIGS. 4A and 4B collectively show a DNA capillary with integrated passageways according to a second embodiment of the present invention, wherein FIG. 4A is an oblique view, and FIG. 4B is a plan view.
FIGS. 5A to 5H are cross sectional views collectively showing how to form a groove by utilizing a semiconductor processing technology for a DNA capillary of the present invention.

### Best Mode of Carrying Out the Invention

### Example 1:

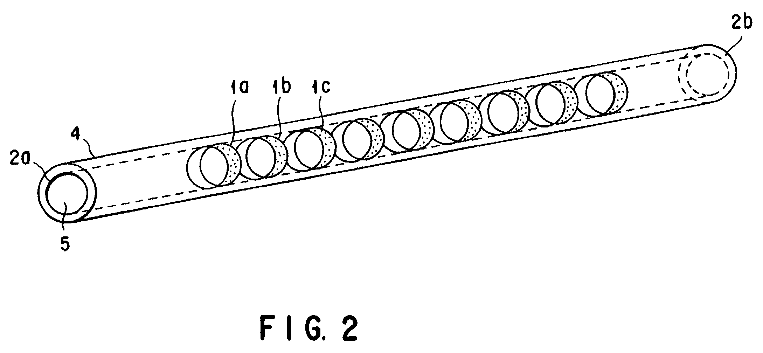

FIG. 2 shows a DNA capillary according to a first embodiment of the present invention. As shown in the drawing, DNA probes 1a, 1b, 1c, etc. differing from each other in kind are immobilized in annular probe regions formed separately from each other on an inner wall 5 of a cylindrical capillary 4 having an injecting open end portion 2a and a discharging open end portion 2b and made of a light transmitting material. It is possible to use the capillary 4 having an inner diameter of about 0.1 mm to about 5 mm. However, the inner diameter of the capillary 4 should desirably be about 0.5 mm to 1 mm to make the capillary 4 easy to handle. The length of the fluid passageway of the capillary 4 should desirably be about 5 mm to about 100 mm. It suffices to determine the inner diameter and the length of the fluid passageway of the capillary 4 depending on the amount of the sample to be measured and the fluidity of the liquid.

The probe immobilized within the capillary 4 consists of a desired base sequence synthesized in advance and should desirably consist of at least 20 bases, preferably 20 to 60 bases, particularly about 40 bases (e.g., 35 to 45 bases). The total number of kinds of the probes immobilized to a single capillary, which differs depending on the object, depends on the number of mRNA molecules to be measured or cDNA molecules converted from the mRNA molecules in a single sample. The number of objects to be measured in the present invention, which are contained in a single sample, ranges from one kind to thousands of kinds. For analyzing the expression pattern of mRNA, which is an object of the present invention, it is necessary to immobilize thousands of kinds of DNA probes. On the other hand, for inspecting, for example, an infectious disease, it suffices to immobilize one to several kinds of DNA probes.

The distance between adjacent DNA probes, e.g., between DNA probes 1a and 1b, should be small where many objects are to be inspected. By contraries, where the number of objects to be measured is small, the distance between adjacent DNA probes can be enlarged. In fact, where a plurality of kinds of DNA probes each having about 20 to 60 bases are arranged for each kind in annular probe regions separated from each other, the DNA probes 1a, 1b, 1c, etc. can be arranged about 1 µm to 5 mm apart from each other. It is also possible to immobilize a plurality of DNA probes of the same kind or to immobilize one or more reference proteins, as desired, leading to an increased diversity of the measurement. In view of the case where traces of object is measured, it is generally desirable to employ a photosensitizing system utilizing fluorescent emission or chemical luminescent emission for the measurement using a DNA capillary. Where the DNA probes 1a, 1b, 1c, etc. are immobilized very close to each other in the capillary 4, it is necessary to read individually a plurality of reaction patterns by using a measuring means having a high resolution such as a fluorescence microscope. On the other hand, where the DNA probes are arranged a large distance, i.e., several millimeters, apart from each other, a naked eye observation can be performed by using a transilluminator. For facilitating the light measurement, it is desirable to use a DNA capillary formed of an optional material excellent in its capability of transmitting light such as plastic materials, silica, glass, polymer or the like. Particularly, it is desirable to use glass or silicon in employing a manufacturing method involving a treatment with silane, which is a step for immobilizing a DNA probe and will be described herein later. If necessary, the DNA capillary may partially reflect or shield light. It is advantageous to use a glass capillary available on the market because the DNA capillary can be manufactured at a low cost. Also, in the case of using a capillary made of a plastic material, it is desirable to use a plastic material having an OH group acting as a means for coupling a DNA probe. In this case, a silane treating agent can be used. However, the plastic material used is not limited to the particular material. Any type of a plastic material can be used as far as the plastic material has a means for coupling a DNA probe.

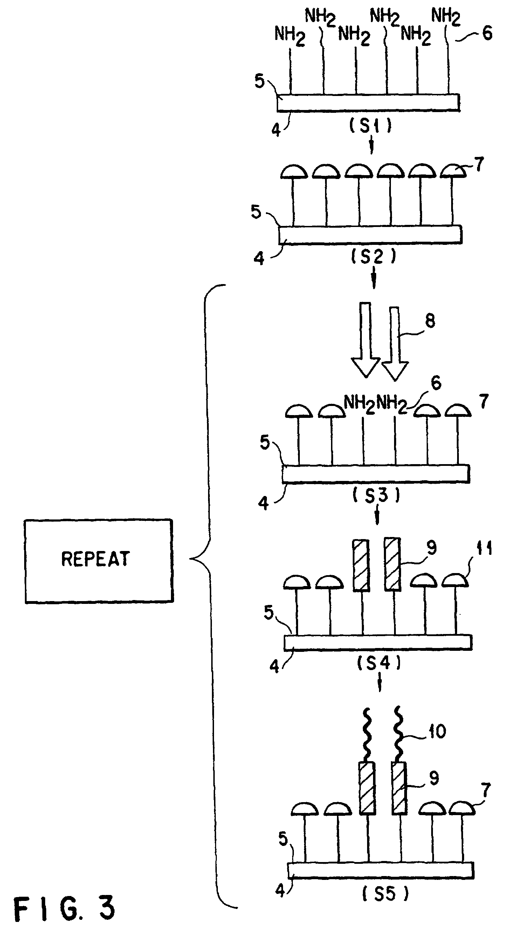

The manufacturing method of a DNA capillary will now be described. FIG. 3 shows how various DNA probes 1a, 1b, 1c, etc. are immobilized on the inner wall 5 of the glass capillary 4. The application of various processing liquids in each manufacturing process can be performed by introduction of the processing liquid through the injecting open end portion 2a of the capillary by a suitable dispensing means and by discharge of the processing liquid through the discharging open end portion 2b by a suitable suction means in the after-treatment. A discharge means such as pipetting and other suitable means can be used as the dispensing means. Also, any type of suction means can be used. It is also possible to employ a natural injection and natural discharge by capillary action.

The manufacturing method will now be described with reference to the scheme shown in FIG. 3. In the first step, a solution containing a silane coupling agent for the silane treatment is applied to the capillary 4 so as to form amino groups (NH₂) on the inner wall 5 (step S1). Then, a solution containing a capping agent 7 which is subjected to photodecomposition upon irradiation with light having a predetermined wavelength, preferably ultraviolet light, is applied to the capillary 4 to allow the capping agent 7 to be coupled with all the amino groups on the surface of the inner wall 5 (step S2). In the next step, the regions in which DNA probes 10 are to be immobilized are selectively irradiated with an ultraviolet light 8 so as to decompose the capping agent 7 and, thus, to expose the amino groups (step S3). The lithography technology employed in the field of semiconductor can be employed for selectively irradiating the desired regions with the ultraviolet light 8. To be more specific, a mask member is used for selectively shielding the ultraviolet light. Alternatively, a lens system can be used for narrowing the range of light irradiation. Since the capillary 4 is cylindrical, the light irradiation can be performed in an optional direction as far as the irradiating light is incident at an angle substantially perpendicular to the side surface of the capillary. Further, since light can be transmitted through the entire region of the capillary 4, the inner wall 5 is deprotected in an annular shape in the region irradiated with the ultraviolet light.

Then, a liquid containing a linker molecule 9 is applied to the capillary 4 (step S4). The linker molecule 9, which is interposed between the amino group 6 bound to the inner wall 5 and the DNA probe 10 so as to link the amino group 6 to the DNA probe 10, has at one end a coupling portion reacting with the amino group to form a bond and at the other end another coupling portion reacting with the amino group or a thiol group to form a bond. The linker molecule 9 applied to the capillary 4 is coupled with the amino group 6 by the coupling portion at one end (step S4). In this step, the coupling portion at the other end is rendered free. Then, a liquid containing the DNA probe 10 having an amino group or thiol group formed at the terminal portion is introduced so as to permit the DNA probe 10 to be coupled with the linker molecule 9 (step S5).

Incidentally, the immobilizing process in each of steps S3, S4 and S5 can be performed while employing a process of washing the inner space of the capillary 4 by using a suitable amount of a washing liquid. Further, a DNA capillary as shown in FIG. 2, i.e., a DNA capillary in which the aimed DNA probes 1a, 1b, 1c, etc. are independently immobilized in the shape of a ring in aimed positions, can be prepared by repeating the above process in another position a desired distance apart from the original position. The term "ring-like" or "annular" shape used herein denotes various shapes such as circular, polygonal and elliptical shapes corresponding to the cross sectional shape of the hollow capillary 4 forming the fluid passageway. Also, the term "fluid passageway" used herein denotes a passageway having a width and a height large enough to allow at least a required amount of the processing liquid to flow therethrough. Further, the fluid passageway is selected to be capable of obtaining the flow promoting function by the capillary action. It follows that a surface simply having 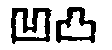 configurations formed thereon is not called the fluid passageway.

The silane coupling agent used in step S1 includes, for example, aminoethyl-aminopropyl-trimethoxy-silane, though other compounds can also be used as the silane coupling agent as far as amino groups can be formed on the surface. For example, it is also possible to use amino silanes such as aminoethyl-aminopropyl-methyl dimethoxy-silane.

The capping agent used in step S2 includes, for example, 4,5-Dimethoxy-2-nitrobenzyl chloroformate, 6-Nitroveratryl chloroformate, 4-Nitrobenzyl chloroformate, and o-Nitrobenzyl-p-nitrophenyl carbonate. However, the capping agent is not limited to these compounds. It is possible to use any substance as the capping agent as far as the substance exhibits an intramolecular cleavage such that the coupling with the amino group can be released upon irradiation with an ultraviolet light or a visible light.

Light having a wavelength of about 350 nm is generally used in step S3. However, light having a wavelength adapted for obtaining an optimum photodecomposition can be selected depending on the kinds of the capping agent and the solvent used. Also, an optical apparatus available on the market can be used for irradiation of a specified light such as an ultraviolet light.

The linker molecule 9 used in step S4 includes, for example, homobifunctional N-hydroxysuccinimidyl (NHS) esters such as Disuccinimidyl suberate and homo-bifunctional imidoesters such as Dimethyladipimidate-2-HCL. The linker molecule used in Example 1 has succinimide groups reacting with the amino group at both terminals of the molecule. However, it is possible for the functional groups at both terminals to differ from each other. For example, it is possible to use a linker molecule having at one terminal an atomic group capable of reaction with a thiol group or a carboxylic group.

A DNA probe having an amino group or a thiol group imparted to the 5' terminal of DNA in the synthesizing step can be used as the DNA probe 10 in step S5. The amino group or thiol group can be imparted easily by using a kit used exclusively for a DNA synthesizer available on the market. A base sequence that can be hybridized with an optional sequence significant in genetics, biochemistry, immunology or pathology can be used in the DNA probe 10, and any sequence can be selected.

The treating conditions in the process of each of steps S1, S2, S3, S4 and D5 relative to the inner wall 5 of the capillary 4 can be determined appropriately depending on the material, shape and size of the capillary used or on the kid of the DNA probe.

For performing the measurement by using the prepared DNA capillary, the processing liquid such as a sample, a reagent, etc. is injected into and discharged from the DNA capillary through the injecting open portion 2a and the discharging open portion 2b. A desired reaction time can be obtained by variously changing the discharge time from the discharging open portion 2b. For example, a sample is introduced through one opening of the capillary 4 so as to carry out reaction at temperatures lower by 10 to 15°C than the melting point (Tm value) of DNA. Then, the capillary is washed with a washing liquid, followed by applying fluorimetry to the sample. A washing liquid having the stringency adjusted by changing appropriately the temperature for use and the composition should desirably be used as the washing liquid. Where the process involved in the measurement of the sample includes a hybridization reaction with the immobilized DNA, the DNA in the sample is made ready for hybridization and, then, is applied to the DNA capillary. Where the biological material itself to be measured is liquid, the biological material can be used as a sample to be measured. Also, a liquid material prepared by dissolving or dispersing the biological material to be measured in a suitable solvent can also be used as a sample to be measured. In short, liquid in an optional state can be used as a sample.

A single capillary is used in Example 1. However, a plurality of capillaries arranged in parallel or bundled together can be processed simultaneously. The plural capillaries, which can be arranged optionally, should desirably be arranged to make the arrangement convenient for a part or entire process involved in the measurement.

In Example 1, a photochemical reaction is performed once for immobilizing the DNA probe. However, it is possible to carry out photochemical reactions a desired number of times, as desired, so as to form DNA probes in the capillary.

### Example 2:

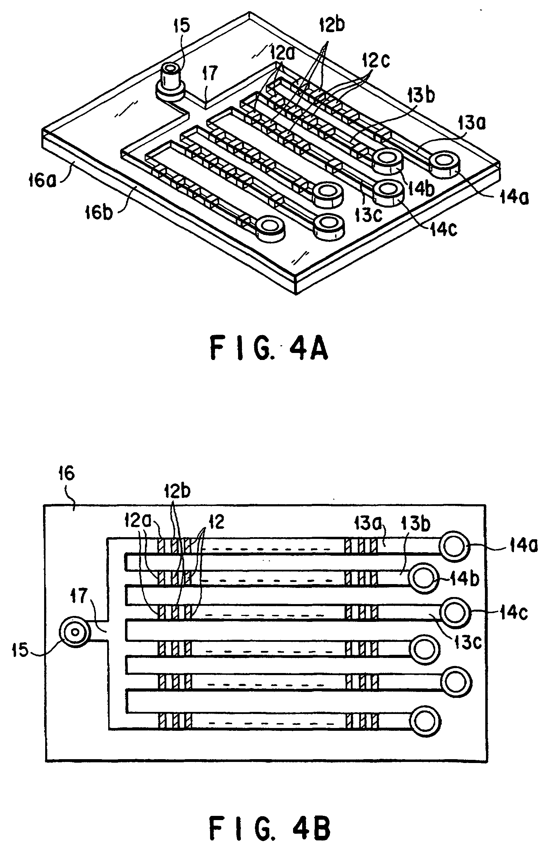

FIG. 4 shows a DNA capillary according to a second embodiment of the present invention. The type shown in FIG. 4 is adapted for efficiently manufacturing a plurality of DNA capillaries. To be more specific, a plurality of fluid passageways can be prepared at once and DNA probes can be immobilized simultaneously on the plural fluid passageways. The DNA capillary in Example 2 is called a DNA capillary array 24 for convenience.

FIG. 4A shows the entire system, and FIG. 4B is a plan view of the DNA capillary system 24. The body of the DNA capillary system 24 comprises a substrate 16 consisting of a lower substrate 16a and an upper substrate 16b bonded to the lower substrate 16a. Each of these lower and upper substrates 16a and 16b is made of glass, silicone, etc. A groove of the pattern as shown in the drawing is formed on the lower substrate 16a. A plurality of DNA capillaries 13a, 13b, 13c, etc. are formed by the,groove. A groove of the pattern similar to that of the lower substrate 16a may also be formed on the upper substrate 16b, too. As shown in FIG. 4B, the ends at one side of the DNA capillaries 13a, 13b, 13c, etc. are positioned right under individual inlet-outlet ports 14a, 14b, 14c, etc. so as to be open to the outer air through the individual inlet-outlet ports 14a, 14b, 14c, etc. These DNA capillaries 13a, 13b, 13c, etc. are combined at the other ends to form a combined fluid passageway 17 extending to reach a region right under a common inlet-outlet port 15 and, thus, are open to the outer air through the common inlet-outlet port 15. These individual inlet-outlet ports 14a, 14b, 14c, etc. and the common inlet-outlet port 15 can be formed by forming thin adhesive layers at predetermined positions by utilizing, for example, a screen printing technology, followed by bonding tubular members such as glass tubes to the adhesive layers. DNA probes 12a, 12b, 12c, etc. are arranged in annular probe regions separated from each other and formed in the fluid passageways of the DNA capillaries 13a, 13b, 13c, etc., as shown in the drawing.

The method employing the photochemical reaction as in Example 1 can also be employed for immobilizing the DNA probes. Example 2 is effective in that a plurality of capillaries can be processed simultaneously. To be more specific, an immobilizing process solution used for immobilizing the DNA probes can be supplied from the common inlet-outlet port 15 to permit the process solution to flow through the combined fluid passageway 17 into the plural DNA capillaries simultaneously. Also, the process solution within the plural DNA capillaries can be discharged simultaneously from the common inlet-outlet ports 15.

Also, the light exposing process for allowing ultraviolet light to decompose the capping agent can be applied simultaneously to the plural capillaries. Specifically, a suitable ultraviolet light irradiating means is arranged above the DNA capillaries. Preferably, the ultraviolet light irradiating means should be of scanning type having a scanning length covering all the plural capillaries and capable of linearly irradiating all the capillaries with ultraviolet light. By moving freely the ultraviolet light irradiating means in an x-direction, i.e., a direction parallel to the fluid passageway in which the DNA probe is immobilized, and/or a Y-direction, i.e., a direction perpendicular to the fluid passageway, so as to scan the DNA capillaries 13a, 13b, 13c, etc., these DNA capillaries are linearly irradiated with ultraviolet light. By this process, the predetermined positions of all the DNA capillaries 13a, 13b, 13c, etc. are linearly irradiated with ultraviolet light. After removal of the capping agent by the irradiation, the further steps described in Example 1 are followed so as to immobilize the DNA probe 12a. In Example 2, it is not absolutely necessary for the lower substrate 16a to transmit light. If at least the upper substrate 16b transmits light, the wall surface in a desired position of fluid passageway formed by the lower substrate 16a and the upper substrate 16b is exposed to light in an annular shape so as to permit the DNA probe to be immobilized in an annular shape conforming with the shape of the light exposure. Then, similar scanning irradiation and immobilization of the DNA probe 12b are performed at a position to which the irradiating means is moved by a predetermined distance in the X-direction parallel to the DNA capillaries 13a, 13b, 13c, etc. so as to immobilize a second kind of DNA probe. The particular operation is repeated so as to form a plurality of DNA regions 12a, 12b, 12c, etc. arranged independent of each other, as shown in FIG. 4.

Where there is a DNA capillary the light exposure of which is desired to be avoided in the locus of scanning in the light exposure process, the light exposure of the particular DNA capillary can be avoided by, for example, selectively switching the irradiation by the ultraviolet light irradiation means by using a suitable switching circuit. In other words, some of the DNA capillaries can be selectively prevented from being irradiated with ultraviolet light in the scanning step. Therefore, it is possible to immobilize DNA probes of different combination for each of the DNA capillaries, leading to diversification of items to be measured. This is advantageous in that it is possible to perform measurement of only the minimum items required in the cases where many items are measured for a single sample and where different items are measured for a plurality of samples. In addition, since unnecessary DNA probes need not be immobilized in the step of preparing the DNA capillaries, the material can be utilized efficiently. It should also be noted that, if DNA probes of the same combination are immobilized in all the DNA capillaries 13, the same measurement can be performed simultaneously on samples the number of which is equal to the maximum number of capillaries.

As described previously, the immobilizing process liquid is introduced through the common inlet-outlet port 15 into the DNA capillaries 13a, 13b, 13c, etc. in the immobilizing process. It is desirable to discharge, particularly, the process liquid containing a DNA probe (see step S5 shown in FIG. 3) and the washing liquid used in the subsequent step through the individual inlet-outlet ports 14a, 14b, 14c, etc. independently. In this case, it is possible to avoid substantially completely the influences of contamination taking place in the case of using a plurality of different kinds of liquid. Also, it is desirable to inject sample liquids, etc. from the individual inlet-outlet ports 14a, 14b, 14c, etc. in measuring the samples. In this case, each liquid can be fluidized in a completely separated state from another liquid, leading to an improved measuring accuracy.

The DNA capillary 13 can be prepared in various sizes depending on the use. In practice, it may suffice for the DNA capillary to have a width of about 10 µm to 5 mm, a depth of about 1 µm to 500 µm, a length of about 5 mm to 100 mm, and a distance between adjacent DNA capillaries of about 10 µm to 5 mm. In general, however, the DNA capillary should desirably have a flattened structure in cross sectional shape having a large width and a small depth in view of the efficiency of reaction because the diffusion rate of cDNA converted from mRNA is low, i.e., about 5 µm/sec. The particular cross sectional shape of the DNA capillary is expected to shorten the reaction time, to diminish the amount of a sample used, and to increase the view field in the observing step.

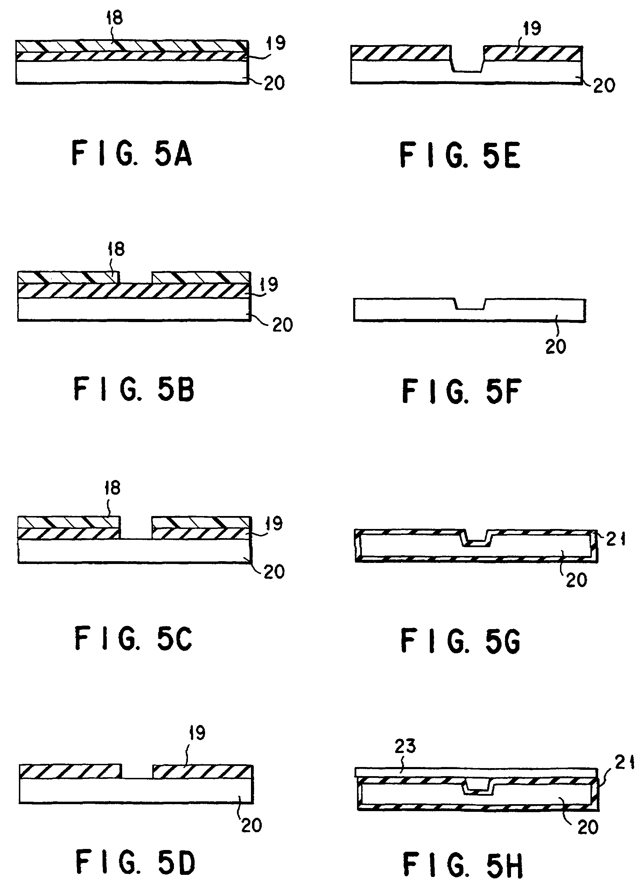

The groove portion of the DNA capillaries 13a, 13b, 13c, etc. can be formed by various methods such as an excimer laser etching and an etching by photolithography. As an example, a groove forming method using a semiconductor processing technology will now be described with reference to the scheme shown in FIG. 5.

In the first step, an oxide film 19 is formed in a thickness of about 5000A on a silicon wafer substrate 20, followed by forming a resist film 18 on the oxide film 19, as shown in FIG. 5A. Then, a mask conforming with a groove pattern on the silicon wafer substrate 20 is prepared, followed by exposing selectively the resist film 18 to light by using an aligner so as to develop the groove pattern, as shown in FIG. 5B. The patterned resist film is used in the next step for etching the oxide film 19, as shown in FIG. 5C. The etching is performed by using an etchant consisting of hydrofluoric acid and ammonium fluoride mixed at a mixing ratio of about 1:9.

Then, the resist film 18 is removed by a method using a mixed solution consisting of sulfuric acid and hydrogen peroxide solution or using an oxygen plasma. After removal of the resist film 18, the silicon wafer substrate 20 is etched by using the patterned oxide film 19, as shown in FIG. 5E. Various known etching methods can be employed in this step including an isotropic or anisotropic wet etching and a dry etching using plasma gas. After the etching of the substrate 20, the oxide film 19 is removed, as shown in FIG. 5F. Since the oxide film 19 is simply removed, it is possible to use in this step a dilute solution, e.g., 50% solution, prepared by diluting, for example, a hydrofluoric acid solution with pure water. Finally, the entire silicon wafer substrate 20 including the groove portion is covered with a silicon oxide film 21, as shown in FIG. 5G.

After the physical processing of the groove as described above, a lid 23 which transmits light is bonded as shown in FIG. 5H so as to prepare the DNA capillary array, in which the individual inlet-outlet ports 14a, 14b, 14c, etc. and the common inlet-outlet port 15 are formed as shown in FIG. 4. The substrate 16 shown in FIG. 4A corresponds to the silicon wafer substrate 20 covered with the oxide film 21 and the lid 23. An anodic bonding method can be employed for the bonding of the lid 23. The anodic bonding method referred to above represents a bonding method in which a voltage of 1,000V is applied between the silicon wafer substrate 20 and the lid 23 while heating the substrate to about 500°C. For employing the anodic bonding method, it is necessary to use the lid 23 made of, for example, pyrex having the thermal expansion coefficient substantially equal to that of silicon. The silane treatment may be performed either before or after the bonding between the silicon wafer substrate 20 and the lid 23. It is not absolutely necessary to apply a silane treatment to the lid 23. However, it is desirable to apply a silane treatment to the lid 23. The silane treatment applied to the lid 23 may be performed either before or after the bonding of the lid 23 to the substrate 20. Where the silane treatment is applied to the lid 23, the DNA probes can be immobilized annularly as shown in FIG. 4, making the silane treatment advantageous in the immobilizing efficiency and the reaction sensitivity. On the other hand, where the silane treatment is not applied to the lid 23, the DNA probes are immobilized by the photochemical reaction in only the groove portion of the silicon wafer substrate 20 so as to obtain U-shaped probe regions. Even in this case, the resultant structure can be used as a DNA capillary array of the present invention.

The silicon wafer substrate 20 is used in the groove formation described above. However, a glass substrate such as a quartz substrate or a pyrex substrate can be used in place of the silicon substrate. In this case, a metal mask such as a gold mask is used in place of the oxide film 19 for the etching of the substrate. Also, the anodic bonding method can be employed for the bonding of the lid 23 to the substrate 20, if a silicon thin film is formed between the substrate 20 and the lid 23. Further, the oxide film 19 used as the etching mask of the silicon wafer substrate 20 can be replaced by, for example, a silicon nitride film or an alumina film.

The DNA capillary array 24 thus prepared produces prominent function and effect as described below. Specifically, since quantities of DNA capillaries can be prepared at once, the manufacturing cost can be lowered. Also, the DNA capillaries can be handled easily. It should also be noted that, since deprotection can be performed in an annular form by irradiating the fluid passageway formed of the capillary with an ultraviolet light, the DNA probe can be efficiently immobilized in an annular form on the inner wall of the capillary so as to improve the measuring sensitivity of the sample. Also, since a plurality of DNA capillaries are formed as an integral structure, the DNA capillary array 24 is advantageous in measuring many samples. For example, different samples can be introduced through the individual inlet-outlet ports 14a, 14b, 14c, etc. and, after biological reactions by incubation for a predetermined time, the samples can be collectively recovered from the common inlet-outlet port 15. Further, the washing liquid and the reagent for measurement can be similarly processed, thereby facilitating the automation and providing an apparatus having a high measurement processing capability.

The DNA capillary of the present invention is not limited to the Examples described above and can be modified in various fashions. For instance, in each of the Examples described above, the injection and discharge of the liquid sample, etc. are performed through different open portions, with the result that each liquid flows in one direction without fail. However, it is possible to allow the liquid to be discharged through the open portion through which the liquid was introduced previously such that the discharge direction is opposite to the injecting direction. Also, in Example 1, a discharge means such as a pipette is used for injecting a liquid for the immobilizing treatment and a liquid for measurement of a sample into the capillary 4. However, other methods can also be employed. For example, a natural injection by capillary force can be achieved by simply dipping the tip portion (injecting open end portion 2a) of the capillary 4 directly in a container containing a required amount of a liquid for the immobilizing treatment or a liquid for the sample measurement. Likewise, a natural discharge can be achieved without using a special sucking device by simply bringing the tip portion (discharging open portion 2b) into contact with a material readily absorbing water such as sponge or a water-absorbing polymer. Therefore, the DNA capillary can be handled easily either manually or automatically. Also, the capillary 4 in Example 1 produces the similar function and effect even if the capillary 4 is used under the state of lying lateral or erected upright. Where the capillary 4 is erected upright such that the injecting open portion 2a occupies the upper portion, the liquid injection can be performed through the upper injecting open portion 2a while discharging the liquid through the lower discharging open portion 2b, and vice versa.

In Example 2, the DNA capillaries 13a, 13b, 13c, etc. arranged in parallel are joined at one side to the combined fluid passageway 17. However, it is also possible to arrange radially these DNA capillaries 13a, 13b, 13c, etc. such that one end portions of these DNA capillaries are joined together at the common inlet-outlet port 15 positioned in the central portion, with the other end portions arranged on a single circular line. Also, if the plural DNA capillaries 13a, 13b, 13c, etc. are arranged to form independent fluid passageways in place of being joined together in the combined fluid passageway 17, it is possible to provide an integrated array which permits processing a plurality of samples efficiently.

The DNA capillary of the present invention can also be used for isolation and purification of DNA or mRNA in addition to the measurement of samples. Also, a protein involved in the antigen-antibody reaction can be used as the probe immobilized in the DNA capillary of the present invention. Further, in measuring the sample, the DNA probe can be selected appropriately from among known DNA probes using optical reaction principles. Also, various reagents required for the measurement such as marker reagents including a fluorescent material, a chemical light-emitting material and a color developing material can be utilized in accordance with the known chemical analytical technology. If necessary, an analytical apparatus available on the market, which is adapted for the selected reaction principle, can be used for automatic measurement.

As described above, the DNA capillary itself forms a fluid passageway in the present invention, making it possible to carry out easily various reactions such as immobilization, measurement and isolation as well as the washing operation. Also, if the DNA capillary of the present invention is connected to a liquid processing apparatus for consecutively processing various liquids, a series of operations can be performed easily. Also, in the case of using a DNA probe in which optional base sequences are synthesized in advance over the entire length of the base sequence, the DNA probe can be immobilized in a single light irradiation, making it possible to markedly suppress the damage done to the DNA probe. It follows that a satisfactory immobilized state can be maintained. Further, since a plurality of kinds of DNA probes are arranged in annular probe regions separately from each other along the fluid passageway, the plural DNA probes can be subjected to a coupling reaction simultaneously and efficiently by simply introducing the sample into the fluid passageway. Still further, since the surface having the DNA probe immobilized thereon is protected inside the capillary, the contamination problem can be eliminated substantially completely. In addition, the DNA capillary can be handled easily.

In Example 2, an optional DNA probe is immobilized in the capillary by a single photochemical reaction, as in Example 1. However, the photochemical reaction can be performed an optional number of times, as desired, so as to prepare a DNA probe having a desired base sequence.

### Industrial Applicability

The DNA capillary provided by the present invention differs from the conventional DNA capillary in that the immobilization of DNA, measurement, etc. can be performed within a closed system, with the result that the DNA capillary of the present invention is strong against contamination and can be handled easily. Also, since a capillary force is utilized in the present invention, the fluid required for the various processing such as various liquids including, for example, a sample and washing liquid, can be handled easily in performing the measurement by utilizing the immobilized DNA probe. Further, if a combined passageway is joined to a plurality of fluid passageways, various processing liquids can be collectively introduced into or recovered from the capillaries through the combined passageway, leading to an increased processing capacity. Still further, the immobilized area within the annular passageway can be increased by applying a photochemical reaction to the light-transmitting fluid passageway, making it possible to perform measurement, etc. efficiently even in the case of using traces of samples. In addition, since a plurality of different DNA probes are immobilized in a single fluid passageway, the measurement can be performed further efficiently. what should also be noted is that a DNA probe having at least 20 bases can be immobilized stably by immobilizing a DNA probe, in which a desired base sequence is synthesized in advance, by a single photoreaction.

## Claims

1. A DNA capillary, comprising:
a fluid passageway having at least a part thereof defined by a wall which transmits light;
a plurality of independent probe regions formed in the inner wall of said fluid passageway; and
DNA probes each immobilized in said probe region, said immobilized DNA probes differing from each other.

2. The DNA capillary according to claim 1, wherein said fluid passageway consists of a hollow capillary having open ends.

3. The DNA capillary according to claim 2, wherein said fluid passageway is cylindrical.

4. The DNA capillary according to any one of claims 1 to 3, wherein said capillary includes a plurality of said fluid passageways arranged to form an integral structure.

5. The DNA capillary according to claim 4, wherein all of said plural fluid passageways are joined at one side to a combined fluid passageway.

6. The DNA capillary according to any one of claims 1 to 5, wherein said DNA probes are arranged independently for each kind in annular probe regions formed apart from each other along the fluid passageway.

7. The DNA capillary according to any one of claims 1 to 6, wherein said fluid passageway is formed by applying an etching treatment to a glass or silicon substrate.

8. The DNA capillary according to any one of claims 1 to 7, wherein said DNA probe is immobilized by utilizing a photochemical reaction.

9. The DNA capillary according to claim 8, wherein said DNA probe is synthesized to have a predetermined base sequence before immobilization and immobilized in said probe region by employing a photochemical reaction once.

## Patentansprüche

1. DNA-Kapillare, umfassend:
einen Fluidkanal, der mindestens teilweise durch eine Wand begrenzt ist, die lichtdurchlässig ist;
eine Vielzahl von unabhängigen Sondenregionen, die in der Innenwand des Fluidkanals ausgebildet sind; und
DNA-Sonden, die jeweils in diesen Sondenregionen immobilisiert sind, wobei die immobilisierten DNA-Sonden sich untereinander unterscheiden.

2. DNA-Kapillare nach Anspruch 1, worin die Fluidkanäle aus einer Hohlkapillare bestehen, deren Enden offen sind.

3. DNA-Kapillare nach Anspruch 2, worin der Fluidkanal zylindrisch ist.

4. DNA-Kapillare nach einem der Ansprüche 1 bis 3, worin die Kapillare eine Vielzahl dieser Fluidkanäle umfaßt, die so angeordnet sind, daß sie eine zusammenhängende Struktur bilden.

5. DNA-Kapillare nach Anspruch 4, worin alle aus dieser Mehrzahl von Fluidkanälen an einer Seite zu einem gemeinsamen Fluidkanal verbunden sind.

6. DNA-Kapillare nach einem der Ansprüche 1 bis 5, worin jede Art von DNA-Sonde unabhängig in ringförmigen Sondenregionen angeordnet ist, die getrennt voneinander entlang des Fluidkanals ausgebildet sind.

7. DNA-Kapillare nach einem der Ansprüche 1 bis 6, wobei der Fluidkanal dadurch ausgebildet wird, daß eine Ätzbehandlung auf einem Glas- oder Siliciumsubstrat durchgeführt wird.

8. DNA-Kapillare nach einem der Ansprüche 1 bis 7, worin die DNA-Sonde mittels einer photochemischen Reaktion immobilisiert wird.

9. DNA-Kapillare nach Anspruch 8, worin die DNA-Sonde so synthetisiert wird, daß sie vor der Immobilisierung eine vorgegebene Basensequenz aufweist, und durch Anwendung einer einmaligen photochemischen Reaktion in der Sondenregion immobilisiert wird.

## Revendications

1. Capillaire d'ADN, comprenant :
une voie de passage de fluide possédant au moins une de ses parties définie par une paroi qui transmet de la lumière ;
une pluralité de régions de sonde indépendantes formées dans la paroi intérieure de ladite voie de passage de fluide ; et
des sondes d'ADN chacune immobilisée dans ladite région de sonde, lesdites sondes d'ADN immobilisées différant les unes des autres.

2. Capillaire d'ADN selon la revendication 1, dans lequel ladite voie de passage de fluide est constituée d'un capillaire creux possédant des extrémités ouvertes.

3. Capillaire d'ADN selon la revendication 2, dans lequel ladite voie de passage de fluide est cylindrique.

4. Capillaire d'ADN selon l'une quelconque des revendications 1 à 3, dans lequel ledit capillaire inclut une pluralité desdites voies de passage de fluide disposées pour former une structure intégrale.

5. Capillaire d'ADN selon la revendication 4, dans lequel la totalité desdites voies de passage de fluide plurales sont raccordées au niveau d'un côté à une voie de passage de fluide combinée.

6. Capillaire d'ADN selon l'une quelconque des revendications 1 à 5, dans lequel lesdites sondes d'ADN sont disposées de façon indépendante pour chaque type dans des régions de sonde annulaires formées séparées les unes des autres le long de la voie de passage de fluide.

7. Capillaire d'ADN selon l'une quelconque des revendications 1 à 6, dans lequel ladite voie de passage de fluide est formée en appliquant un traitement d'attaque à un substrat de verre ou de silicium.

8. Capillaire d'ADN selon l'une quelconque des revendications 1 à 7, dans lequel ladite sonde d'ADN est immobilisée en utilisant une réaction photochimique.

9. Capillaire d'ADN selon la revendication 8, dans lequel ladite sonde d'ADN est synthétisée pour avoir une séquence de bases prédéterminée avant l'immobilisation et immobilisée dans ladite région de sonde en employant une réaction photochimique une fois.
